# EUROPEAN PATENT SPECIFICATION

(11) **EP 3 981 004 B1**
(45) Date of publication and mention of the grant of the patent: **13.08.2025**
(21) Application number: 20751852.3
(22) Date of filing: 08.06.2020
(51) Int. Cl.: G16H 20/10, G16H 50/70

(54) **MISSED-BOLUS DOSE DETECTION AND RELATED SYSTEMS, METHODS AND DEVICES**
ERKENNUNG VERPASSTER BOLUSDOSEN UND VERWANDTE SYSTEME, METHODEN UND GERÄTE
LA DÉTECTION DES DOSES DE BOLUS MANQUÉES ET LES SYSTÈMES, MÉTHODES ET DISPOSITIFS CONNEXES

(30) Priority: 10.06.2019 US 201962859624 P
(43) Date of publication of application: 13.04.2022
(60) Divisional application: 25170980.4
(73) Proprietor: Bigfoot Biomedical, Inc., Milpitas, CA 95035 (US)
(72) Inventor: CONSTANTIN, Alexandra Elena, Milpitas, California 95035 (US); EGHTESADI, Zahra, Milpitas, California 95035 (US)
(74) Representative: Murgitroyd & Company
(86) International application number: PCT/US2020/070127
(87) International publication number: WO 2020/252496

(56) References cited:
- US-A1- 2007 016 170
- US-A1- 2013 053 819
- US-B2- 9 901 677
- ANONYMOUS: "Statistical classification - Wikipedia", 28 May 2018 (2018-05-28), XP055724678, Retrieved from the Internet <URL:https://en.wikipedia.org/w/index.php?title=Statistical_classification&oldid=843354472> [retrieved on 20200824]
- ANONYMOUS: "Synthetic data - Wikipedia", 26 March 2019 (2019-03-26), XP055724727, Retrieved from the Internet <URL:https://en.wikipedia.org/w/index.php?title=Synthetic_data&oldid=889574974> [retrieved on 20200824]

## Description

### TECHNICAL FIELD

Disclosed embodiments relate, generally, to missed-bolus dose detection, and more specifically, to retrospectively detecting missed-bolus dosing related to insulin therapy.

### BACKGROUND

Diabetes mellitus is a chronic metabolic disorder caused by the inability of a person's pancreas to produce sufficient amounts of the hormone insulin such that the person's metabolism is unable to provide for the proper absorption of sugar and starch. The inability to absorb those carbohydrates sometimes leads to hyperglycemia, i.e., the presence of an excessive amount of glucose within the blood plasma. Hyperglycemia has been associated with a variety of serious symptoms and life threatening long-term complications such as dehydration, ketoacidosis, diabetic coma, cardiovascular diseases, chronic renal failure, retinal damage and nerve damages with the risk of amputation of extremities.

Because healing is not yet possible, a permanent therapy is necessary which maintains a proper blood glucose level within normal limits. Maintaining a proper glucose level is achieved by regularly supplying insulin to a person with diabetes (PWD). Maintaining a proper blood glucose level creates a significant cognitive burden for a PWD and affects many aspects of the PWD's life. For example, the cognitive burden on a PWD can be attributed to, among other things, tracking meals and constant check-ins and minor course corrections of blood glucose levels. The adjustments of blood glucose levels by a PWD can include taking insulin, tracking insulin dosing and glucose, deciding how much insulin to take, how often to take it and how to time insulin doses in relation to meals and/or glucose fluctuations. These factors make up just a portion of the significant cognitive burden of a PWD.

From the moment a PWD wakes up to the moment they go to bed, a PWD is constantly checking their blood glucose level, considering the amount and type of food they have and will eat, considering how much active insulin is still in their body, trying to anticipate future insulin requirements, checking and rechecking their supplies, and confirming that their equipment is still working.

Insulin-based management of diabetes requires significant attention to detail throughout the day. Even with careful planning and self-monitoring, a PWD may skip doses, double dose, or dose the wrong amount and/or type of insulin. As mentioned already, insufficient insulin can result in hyperglycemia, and too much insulin can result in hypoglycemia, which can result in clumsiness, trouble talking, confusion, loss of consciousness, seizures, or death.

Document US 2013/053819 A1 discloses receiving and storing data indicative of a user's blood glucose levels, i.e. therapy data associated with insulin based management of the user's diabetes, identification of a retrospective time period of and based on the user's blood glucose levels for said time period the determination whether the user missed a bolus or not, i.e. a classification (bolus missed or not), and according labeling of the time period, i.e. storing information whether a bolus was missed during said time period or not. **It** does not disclose a trained missed-bolus classifier based on training data and feature sets, wherein the training data comprises positive class training data formed from positive chunked data and negative class training data formed from negative chunked data, and wherein the feature sets comprises aggregated feature set values for the positive class training data formed from feature values for positive class chunked data and aggregated feature set values for the negative class training data formed from feature values for negative class chunked data.

### BRIEF DESCRIPTION OF THE DRAWINGS

While this disclosure concludes with claims particularly pointing out and distinctly claiming specific embodiments, various features and advantages of embodiments within the scope of this disclosure may be more readily ascertained from the following description when read in conjunction with the accompanying drawings, in which:
FIG. 1 shows a simplified block diagram of a computing platform for detecting a missed bolus, in accordance with one or more embodiments.
FIG. 2 shows a flowchart of a process for detecting a missed-bolus, in accordance with one or more embodiments.
FIG. 3 shows a diagram of an example report of retrospective studies created by a computing platform of FIG. 1, in accordance with one or more embodiments.
FIG. 4 shows a diagram of an example report of retrospective studies created by a computing platform of FIG. 1, in accordance with one or more embodiments.
FIG. 5 shows a simplified block diagram of a system for insulin-based management of diabetes, in accordance with one or more embodiments.
FIG. 6 shows a functional block diagram of a system for training a missed-bolus classifier using machine learning, in accordance with one or more embodiments.
FIG. 7 shows a flowchart of a process for training a missed-bolus classifier using machine learning, in accordance with one or more embodiments.
FIG. 8 illustrates a data journey in accordance with one embodiment.

### DETAILED DESCRIPTION

The following description provides specific details to provide a thorough description of various embodiments of the invention. However, one of ordinary skill in the art will understand that the disclosed embodiments may be practiced without using these specific details. Indeed, the disclosed embodiments may be practiced in conjunction with conventional systems and methods used in the industry. In addition, only those elements helpful to understand and enable one of ordinary skill in the art to practice the disclosed embodiments are described in detail. One of ordinary skill in the art will recognize that some elements not described herein but, using various conventional method components and acts, would be in accord with the embodiments of this disclosure.

The following description may include examples to help enable one of ordinary skill in the art to practice the disclosed embodiments. The use of the terms "exemplary," "by example," "for example," "e.g.," and "such as" means that the related description is explanatory. The use of such terms is not intended to limit the scope of an embodiment or this disclosure to the specified components, steps, features, functions, arrangement of components, or the like. Moreover, the use of such terms does not indicate or imply that the related description comprises, or is, a preferred embodiment.

Any drawings accompanying this disclosure are for illustrative purposes only, and no scale is intended unless specifically indicated. Elements common among figures may retain the same numerical designation; however, the similarity in numbering does not mean that the structures or components are necessarily identical in size, composition, configuration, or any other property.

It will be readily understood that the components of the embodiments as generally described herein and illustrated in the drawing could be arranged and designed in a wide variety of different configurations. Thus, the following description of various embodiments is not intended to limit the scope of the present disclosure, but is merely representative of various embodiments. While the various aspects of the embodiments may be presented in drawings, the drawings are not necessarily drawn to scale unless specifically indicated.

As noted, above, specific implementations shown and described are only examples and should not be construed as the only way to implement the present disclosure unless specified otherwise herein. Elements, circuits, and functions may be shown in block diagram form in order not to obscure the present disclosure in unnecessary detail. Block definitions and partitioning of logic between various blocks is/are examples of a specific implementation. It will be readily apparent to one of ordinary skill in the art that the present disclosure may be practiced by numerous other partitioning solutions. For the most part, details concerning timing considerations and the like have been omitted where such details are not necessary to obtain a complete understanding of the present disclosure and are within the abilities of persons of ordinary skill in the relevant art.

Many of the functional units described in this specification may be illustrated, described or labeled as logic, modules, engines, threads, or other segregations of programming code, to more particularly emphasize their implementation independence in accomplishing the features, functions, tasks or steps that are generally described herein. The various illustrative logical blocks, modules, and circuits described in connection with the embodiments disclosed herein may be at least partially implemented or performed with a general purpose processor, a special purpose processor, a Digital Signal Processor (DSP), an Application Specific Integrated Circuit (ASIC), a Field Programmable Gate Array (FPGA) or other programmable logic device, discrete gate or transistor logic, discrete hardware components, or any combination thereof designed to perform the functions described herein.

The functional units may be implemented using software or firmware, stored on a computer-readable storage medium, in system memory, or a combination thereof for execution by various types of processors. Some examples of languages that may be used to write the software include, but are not limited to, an extensible markup language, C, C++, JAVA, MATLAB, MINITAB, EXPRESS, DRAKON, DYNA, PYTHON, MOOSE, and RUBY. The software programs may be further translated into machine language or virtual machine instructions and stored in a program file in that form. The program file may then be stored on or in one or more of the articles of manufacture. Although enabling software might be "written on" a disc, "embodied in" an integrated circuit, "carried over" a communications circuit, "stored in" a memory chip, or "loaded in" a cache memory, it will be appreciated that, for the purposes of this application, the software is referred to simply as being "in" or "on" the computer readable medium. Thus, the terms "in" or "on" are intended to encompass the above mentioned and all equivalent and possible ways in which software can be associated with a computer readable medium.

In the case of a general-purpose computer, these logic and modules may be embodied in software classes and applications executed by processor cores, and while the modules are executing the general-purpose computer may be thought of as a special purpose computer or a specific purpose computer. The logic and modules may also relate to specific purpose hardware, including the firmware and machine code, controlling its operation. An identified module of executable code may, for instance, comprise one or more physical or logical blocks of computer instructions, which may, for instance, be organized as a thread, object, procedure, or function. Nevertheless, the executable code of an identified module need not be physically located together, but may comprise disparate instructions stored in different locations which, when joined logically together, comprise the module and achieve the stated purpose for the module.

A module of executable code may comprise a single instruction, or many instructions, and may even be distributed over several different code segments, among different programs, and across several storage or memory devices. Similarly, operational data may be identified and illustrated herein within modules, and may be embodied in any suitable form and organized within any suitable type of data structure. The operational data may be collected as a single data set or may be distributed over different locations including over different storage devices, and may exist, at least partially, merely as electronic signals on a system or network. Where a module or portions of a module are implemented in software, the software portions are stored on one or more physical devices, which are referred to herein as computer-readable media.

In some embodiments, the software portions are stored in a non-transitory state such that the software portions or representations thereof, persist in the same physical location for a period of time. Additionally, in some embodiments, the software portions are stored on one or more non-transitory storage mediums, which include hardware elements capable of storing non-transitory states and/or signals representative of the software portions, even though other portions of the non-transitory storage mediums may be capable of altering and/or transmitting the signals. Examples of non-transitory storage mediums are flash memory and certain types of random-access memory (RAM). Another example of a non-transitory storage medium includes a read-only memory (ROM) which can store signals and/or states representative of the software portions for a period of time. However, the ability to store the signals and/or states is not diminished by further functionality of transmitting signals that are the same as, or representative of, the stored signals and/or states. For example, a processor may access the ROM to obtain signals that are representative of the stored signals and/or states to execute the corresponding software instructions.

A general-purpose processor may be a microprocessor, but in the alternative, the processor may be any conventional processor, controller, microcontroller, or state machine. A processor may also be implemented as a combination of computing devices, such as a combination of a DSP and a microprocessor, a plurality of microprocessors, one or more microprocessors in conjunction with a DSP core, or any other such configuration. A general-purpose computer including a processor is considered a special-purpose computer when the general-purpose computer is configured to execute computing instructions (e.g., software code) related to embodiments of the present disclosure.

The embodiments disclosed herein may be described in terms of a process that is depicted as a flowchart, a flow diagram, a structure diagram, or a block diagram. Although a flowchart may describe operational acts as a sequential process, many of these acts can be performed in another sequence, in parallel, or substantially concurrently. In addition, the order of the acts may be rearranged. A process may correspond to a method, a thread, a function, a procedure, a subroutine, a subprogram, etc. Furthermore, the methods disclosed herein may be implemented in hardware, software, or both. If implemented in software, the functions may be stored or transmitted as one or more instructions or code on computer-readable media. Computer-readable media includes both computer storage media and communication media including any medium that facilitates transfer of a computer program from one place to another.

Various embodiments described herein may be described as implemented in or by a "computer," "computing system," or a "computing platform," which are to be understood to include at least one non-transitory computer-readable medium and at least one processing unit. In general, the storage medium will store, at one time or another, at least portions of an executable program code, and a processor(s) will execute one or more of the instructions included in that executable program code.

It will be appreciated that the term "executable program code" and the term "software" mean substantially the same thing for the purposes of this description. It is not necessary to the practice of the various embodiments described herein that the storage medium and the processing unit be physically located in the same place. That is to say, it is foreseen that the processor and the memory might be distributed among physical pieces of equipment or even in geographically distinct locations. One of ordinary skill in the art will appreciate that "media," "medium," "storage medium," "computer-readable media," or "computer-readable medium" as used herein, may include a diskette, a magnetic tape, a digital tape, a compact disc, an integrated circuit, a ROM, a CD, DVD, Blu-Ray, a cartridge, flash memory, a PROM, a RAM, a memory stick or card, or any other non-destructive storage medium useable by computers, including those that are re-writable.

Disclosed embodiments may be performed, in whole or in part, in cloud computing, client-server, or other networked environments, or any combination thereof. One or more components of such systems (e.g., a computing platform) may be located in a singular "cloud" or network, or spread among many clouds or networks. End-user knowledge of a physical location and/or configuration of components of a computing platform is not required. Moreover, components of such systems may be operatively linked via one or more electronic communication links. Such electronic communication links may be established, at least in part, via a network such as the Internet and/or other networks. It will be appreciated that this is not intended to be limiting, and that the scope of this disclosure includes embodiments in which servers, clients, computing platforms, and/or external resources may be operatively linked via some other communication media.

Users may interact with the computing platforms described herein by way of graphical user interfaces (GUIs) on a display and input devices such as touchscreens, keyboards, a computer mouse, touchpads, buttons, switches, jumpers, and the like. A GUI may include a console and/or dashboard and a user may interact with the GUI and, in turn, underlying software applications.

Any reference to an element herein using a designation such as "first," "second," and so forth does not limit the quantity or order of those elements, unless such limitation is explicitly stated. Rather, these designations may be used herein as a convenient method of distinguishing between two or more elements or instances of an element. Thus, a reference to first and second elements does not mean that only two elements may be employed there or that the first element must precede the second element in some manner. In addition, unless stated otherwise, a set of elements may comprise one or more elements.

As used herein, the term "substantially" about a given parameter, property, or condition means and includes, to a degree, that one of ordinary skill in the art would understand that the given parameter, property, or condition is met with a small degree of variance, such as, for example, within acceptable manufacturing tolerances. By way of example, depending on the parameter, property, or condition that is substantially met, the parameter, property, or condition may be at least 90% met, at least 95% met, or even at least 99% met.

When a bolus dose (the terms "bolus dose" and "bolus dose of insulin" are used interchangeably in this disclosure) is missed, a PWD is at risk for elevated blood glucose and hyperglycemia. Moreover, in order to administer a late bolus dose, a PWD should consider that some glucose may already have been acted on by existing insulin in their body. If the PWD administers the same amount as the original bolus dose, some of the insulin may be active in the body for a long time. If the catchup bolus dose is large enough, there is a risk of causing hypoglycemia, either from that dose or a later dose due to insulin stacking. So, a PWD carries the cognitive burden of remembering to administer a bolus dose, and if they forget, the additional cognitive burden of a more difficult calculation for a catchup bolus dose.

Bolus calculators (typically a worksheet or software application) can relieve some of the cognitive burden and, in theory, reduce risks associated with incorrectly calculating a catch-up bolus dose of insulin. However, bolus calculators are not always accurate, and PWDs and their caregivers do not always provide correct data. So, bolus calculators can provide a false sense of security that results in relaxed vigilance and more missed bolus doses.

If a health care provider (HCP) is aware that their patient frequently misses bolus-doses then the HCP can take corrective action or initiate corrective action by their patient. For example, an HCP can educate a patient about risks associated with missed bolus doses, propose habits and strategies for remembering to timely administer a bolus dose, increase the amount of long acting (LA) insulin a PWD administers each day, and even change the PWDs insulin delivery mechanism (e.g., from an insulin pen to an insulin pump with continuous glucose monitor).

The inventors of this disclosure appreciate a need for detection of missed bolus doses. In particular, a need for detecting, retrospectively, that a bolus dose or bolus doses were missed, and in some implementations the frequency at which bolus doses were missed.

In this disclosure, the terms "retrospective" and "retrospectively" have the same meanings as commonly understood by one of ordinary skill in the technical art to which this disclosure belongs.

In this disclosure, the term "insulin therapy" means insulin-based management of diabetes. Unless otherwise indicated, when "therapy" is used herein it should be understood to mean "insulin therapy." For example, "therapy data" should be understood to mean "insulin therapy data" and "therapy management system" should be understood to mean "insulin therapy management system."

Some disclosed embodiments relate, generally, to performing a process for detecting a missed-bolus dose using therapy data, the therapy data being associated with an insulin-based management of a person's diabetes over a period of time. In one embodiment, a retrospective time period is identified during (i.e., within) a period of time of the insulin therapy to which the therapy data relates. As a non-limiting example, a retrospective time period may be defined by a timestamp or series of timestamps that are within the retrospective time period. A classifier assigns a label to the retrospective timestamp as either detecting or not detecting a missed-bolus event. In one embodiment, a classifier is a trained model (i.e., obtained using machine learning techniques).

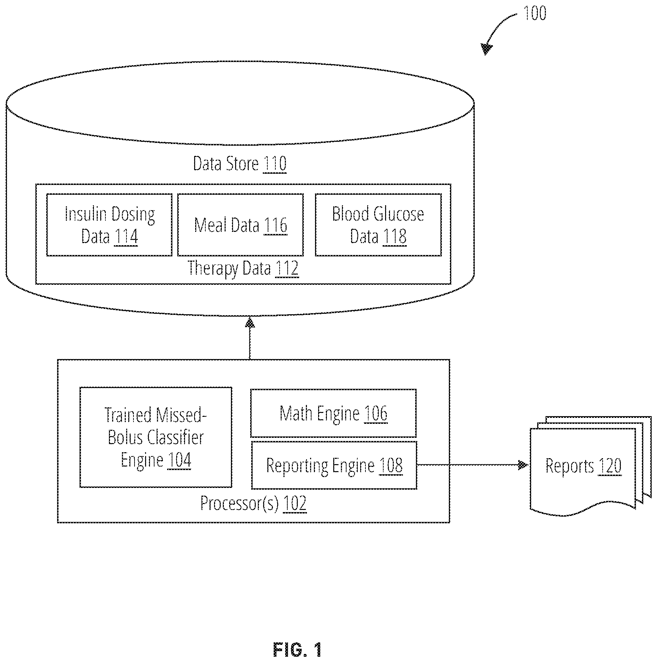

FIG. 1 shows a computing platform 100 for detecting a missed bolus event, in accordance with disclosed embodiments. In disclosed embodiments, computing platform 100 is, or is operative to be executed as, a data processing system, and more specifically, as a data processing system for performing retrospective missed-bolus detection. Computing platform 100 may include data store 110 and processor(s) 102. Data store 110 may include therapy data 112. Therapy data 112 is data related to insulin therapy of one or more persons. In the embodiment shown in FIG. 1, therapy data 112 includes insulin dosing data 114, meal data 116, and blood glucose data 118. Alternatively or additionally, therapy data 112 may include exercise data, sleep data, and/or physiological parameters of a patient (e.g., an insulin sensitivity factor or insulin-to-carbohydrate ratio).

Blood glucose data 118 may include data about blood glucose in a human body at one or more times. Blood glucose data 118 may include measurements of blood glucose levels, for example, raw blood glucose measurements, blood glucose estimates based on blood glucose measurements, and/or aggregations of the same (e.g., averages, trends and/or metrics). Blood glucose data 118 may include date and time (e.g., a timestamp), and a value for each blood glucose measurement. In disclosed embodiments, any suitable glucose sensor may provide blood glucose data 118, for example, a continuous glucose monitor (CGM), a flash glucose monitor, a blood glucose meter (BGM). In the case of CGMs and flash glucose monitors, they may be configured to provide blood glucose data 118 based on interstitial fluid glucose levels of a person, which may be correlated to blood glucose levels. A BGM may be configured to provide blood glucose data based on a blood sample. Accordingly, the term "blood glucose" is not limited herein to using just blood glucose data, values, levels etc., but is also intended to include interstitial fluid glucose levels, intermediate measurements, and legal equivalents thereof.

Insulin dosing data 114 may include dosing event data. Dosing event data may include data about insulin dosing actions at one or more times and may include, for example, a dosing time or time range, type of insulin (e.g., LA insulin and rapid acting (RA) insulin) dosed, brand of insulin, and/or amount of dosed insulin. In some embodiments, dosing event data may include an indication of a dosing mechanism, for example, injection pen, inhaler, or infusion pump. In some embodiments, dosing event data may include an indication of whether dosing event data, in part or in whole, is based on an actual dosing action (e.g., detecting insulin delivery, for example, based on a manual action of a pump or a control signal configured to cause insulin delivery), user tracking of dosing actions (e.g., a PWD or caregiver enters a dose using a therapy application executing on a mobile device), or inferred dosing actions (e.g., from capping/uncapping of an injection pen).

Processor(s) 102 may be configured to execute a number of engines for performing disclosed embodiments. In the embodiment shown in FIG. 1, processor(s) 102 includes trained missed-bolus classifier engine 104, math engine 106, and reporting engine 108.

Trained missed-bolus classifier engine 104 may be configured, generally, to process therapy data 112 or part(s) of therapy data 112, and detect one or more missed boluses. In one embodiment, a retrospective time period may be defined (e.g., as a setting), and a part of therapy data 112 processed by the trained missed-bolus classifier engine 104 may correspond to the retrospective time period. In one embodiment, trained missed-bolus classifier engine 104 may be a binary classifier, that is, return one of two results, a first result corresponding to "missed bolus detected" and a second result corresponding to "no missed bolus detected." Trained missed-bolus classifier engine 104 may assign only one label to each retrospective timestamp, for example, "missed bolus detected" and "no missed bolus detected."

In disclosed embodiments, trained missed-bolus classifier engine 104 may be trained using one or more supervised and/or unsupervised learning techniques, including those described in more detail in this disclosure.

Math engine 106 may be configured to perform various statistical calculations using therapy data 112 and results provided by trained missed-bolus classifier engine 104. In various embodiments, statistical calculations may include, for example, frequency calculations, confidence calculations, probability calculations, and more.

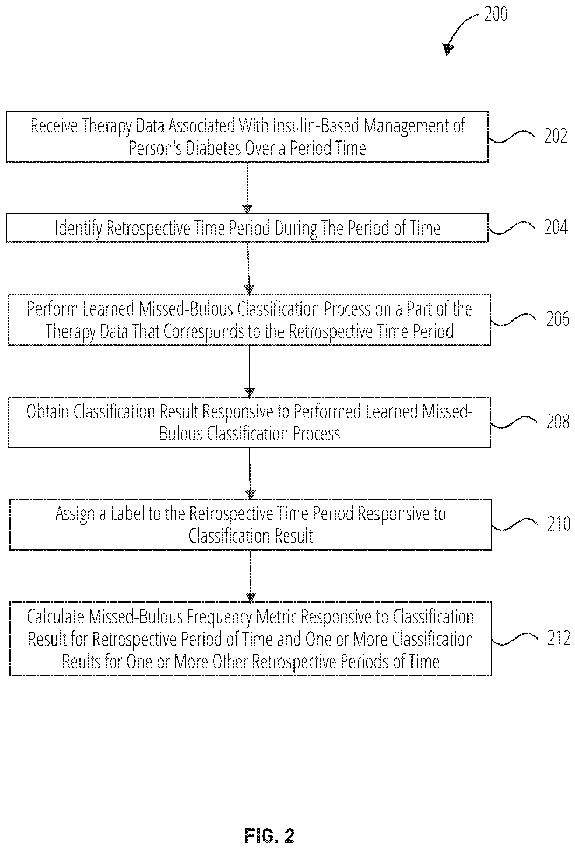

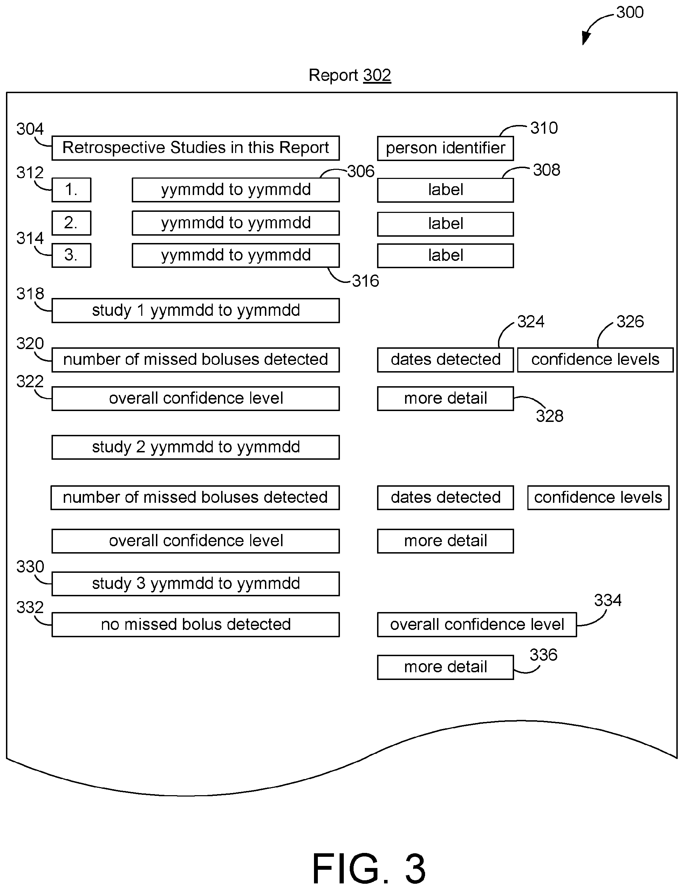

Reporting engine 108 may be configured, generally, to generate one or more reports 120 responsive to trained missed-bolus classifier engine 104 and/or math engine 106. Reports 120 may include descriptions of retrospective studies performed at computing platform 100 as more fully described with reference to FIG. 2 and FIG. 3, and may include, for example, patient identifiers, descriptions of retrospective time periods, assigned class labels, the class labels and more.

FIG. 2 shows a flowchart of a process 200 for detecting a missed bolus, in accordance with one or more embodiments. Process 200 may be performed for example, in whole or in part, by embodiments disclosed herein, including by computing platform 100. In operation 202, process 200 receives therapy data associated with an insulin-based management of a person's diabetes over a period of time. In operation 204, process 200 identifies a retrospective time period during the period of time. In one embodiment, the retrospective time period may be identified based on a setting associated with performing process 200. For example, a user may set one or more retrospective time periods, including the retrospective time period used in operation 204 prior to the current instance of performing process 200. In operation 206, process 200 performs a missed-bolus classification process on a part of the therapy data that corresponds to the retrospective time period. In operation 208, process 200 obtains a classification result responsive to the performed missed-bolus classification process. In one embodiment, a classification result may be binary, e.g., "missed bolus detected" or "no missed bolus detected." In one embodiment, a classification result may be indicative of a number of missed-bolus doses detected in the retrospective time period. In operation 210, process 200 assigns a label to the retrospective time period responsive to the classification result, e.g., "missed bolus detected" or "no missed bolus detected." In operation 212, process 200 calculates a missed-bolus frequency metric responsive to the classification result for the retrospective period of time and one or more classification results for one or more other retrospective periods of time.

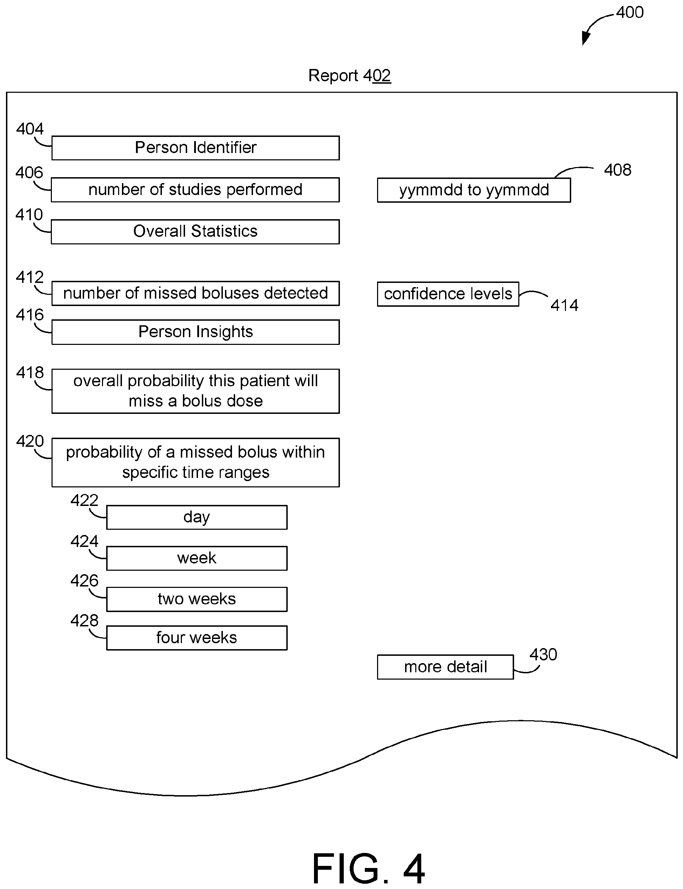

Some embodiments relate, generally, to reporting of missed boluses detected in accordance with disclosed embodiments. FIG. 3 and FIG. 4 show report 302 and report 402, respectively, which are examples of reports that may be created by computing platform 100 of FIG. 1 for retrospective studies. In one embodiment, report 302 and/or report 402 may be a computer file including one or more of the fields shown in FIG. 3 and FIG. 4, respectively. In one embodiment, report 302 and report 402 may include an electronic document in a human-readable form, a machine-readable form, or both human-readable and machine-readable forms. In disclosed embodiments, a "computer file" refers to a computer resource for recording data discretely in a computer storage device and to a stream of data received on a tangible computer medium. In disclosed embodiments, "data" refers to both data and information.

Turning to FIG. 3, report 302 includes a list 304 of retrospective studies included in report 302. In this example, there are entries for three studies associated with a person identified at identifier 310. Entry 312 for the first study includes a date range 306 and a label 308. The other entries have the same elements. Date range 306 corresponds to a retrospective time period processed by trained missed-bolus classifier engine 104. Label 308 corresponds to a label assigned by trained missed-bolus classifier engine 104 to the retrospective timestamps corresponding to date range 306.

For each entry, additional data may be provided. In this example, for entry 312, additional data 318 is provided in report 302. Additional data 318 includes fields for number of missed boluses detected 320, overall confidence level 322, dates detected 324, and confidence levels 326 for dates detected 324. Number of missed boluses detected 320 describes the overall number of missed bolus doses that were detected by trained missed-bolus classifier engine 104 for date range 306. Overall confidence level 322 describes a calculated confidence level that the overall number of missed bolus doses that were detected were true missed bolus doses. Dates detected 324 is a list of dates for which at least one missed bolus was detected by trained missed-bolus classifier engine 104. In one embodiment, a number of missed bolus doses for each date may be included for each date in dates detected 324. Confidence levels 326 describes calculated confidence levels for each respective date in dates detected 324. Additionally or alternatively, overall confidence level 322 and confidence levels 326 may indicate confidence that trained missed-bolus classifier engine 104 detected all missed-boluses.

For entry 314, additional data 330 is provided in report 302. In this example, no missed bolus was detected in the study corresponding to entry 314, so additional data 330 includes fields for no missed bolus detected 332. Additional data 330 also includes an overall confidence level 334 that describes a calculated confidence level that there were no true missed bolus doses in the date range 316.

In some embodiments, supporting data may be provided for any of the data in additional data 318 and/or additional data 330. Supporting data for additional data 318 and additional data 330 may be included in more detail 328 and more detail 336, respectively.

Turning to FIG. 4, data in report 402 may be created in addition to, or alternatively to, that in report 302. In the embodiment shown in FIG. 4, report 402 includes fields for data about a patient for which trained missed-bolus classifier engine 104 performed retrospective missed-bolus detection in accordance with disclosed embodiments. Report 402 includes fields for: person identifier 404, number of studies performed 406, and dates ranges 408 for which studies were performed. Report 402 also includes fields for overall statistics 410 and person insights 416.

Overall statistics 410 are statistical observations related to missed-bolus detection for the person identified in person identifier 404. In the embodiment shown in FIG. 4, overall statistics 410 includes fields for number of missed boluses detected 412 and overall confidence levels 414 that the number of missed boluses detected 412 correspond to true missed boluses. Additionally or alternatively, confidence levels 414 may indicate confidence that trained missed-bolus classifier engine 104 detected all missed boluses.

Person insights 416 are observations about predicted behaviors related to missed-bolus dosing of a person associated with person identifier 404. In the embodiment shown in FIG. 4, person insights 416 includes fields for overall probability this patient will miss a bolus dose 418 and probability of a missed bolus within specific time ranges 420. Overall probability this patient will miss a bolus dose 418 is a probability that a person associated with person identifier 404 will miss a bolus dose during insulin therapy. Probability of a missed bolus within specific time ranges 420 includes probabilities that a person associated with person identifier 404 will miss a bolus dose during insulin therapy within a specific time range. In the embodiment shown in FIG. 4, probabilities are provided for time ranges of a day 422, a week 424 two weeks 426, and four weeks 428. Fields for supporting data related to data in report 402 may be included, such as more detail 430.

Any suitable technique used by one of ordinary skill in the art in the field of data science to calculate and/or express probabilities may be used with disclosed embodiments.

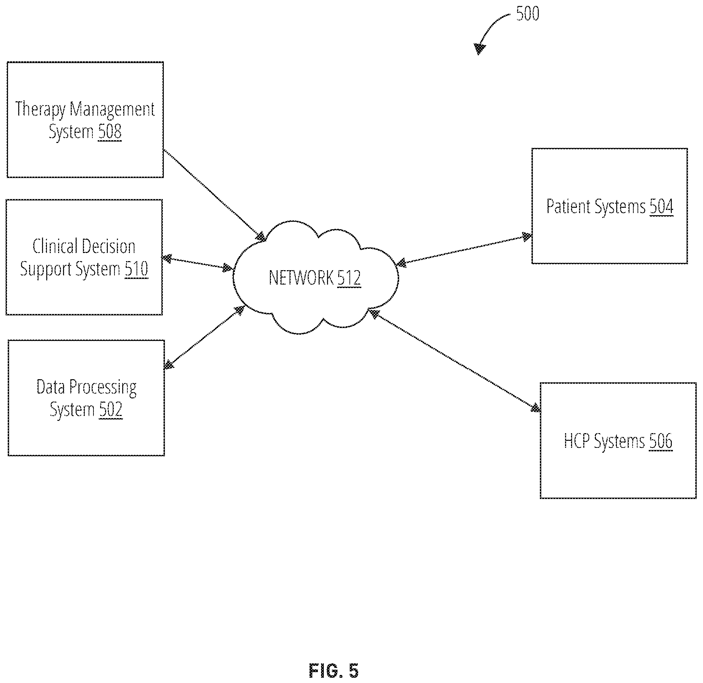

Some embodiments relate, generally, to insulin therapy systems and elements thereof that incorporate systems, methods and devices for missed-bolus detection. FIG. 5 shows a system 500 for insulin therapy, in accordance with disclosed embodiments. In the embodiment shown in FIG. 5, data processing system 502, clinical decision support system 510, and therapy management system 508 are computing platforms configured, generally, to provide various services related to insulin therapy, in whole or in part, to each other and to HCP systems 506 and patient systems 504. HCP systems 506 may include, for example, portals, dashboards, electronic medical record systems, computing platforms executing the same, and more.

In disclosed embodiments, therapy management system 508 may be one or more computing platforms configured to receive and store therapy data (such as therapy data 112 in FIG. 1) and physiological parameters about patients, issue alarms and alerts, and manage therapy settings for insulin delivery systems - all related to insulin-based management of a PWD's diabetes.

In disclosed embodiments, clinical decision support system 510 may be one or more computing platform configured as a health data technology system for assisting HCPs with clinical decision-making tasks, and more specifically in this example, assist HCPs with clinical decision-making tasks related to a PWD's insulin therapy. In disclosed embodiments, clinical decision support system 510 is configured to assist with insulin-based management of diabetes, and automatically analyzes therapy data 112 (FIG. 1), identifies clinically relevant patterns in a PWD's therapy from therapy data 112, and provides data and recommendations to HCP systems 506 based on those patterns. A goal of embodiments of clinical decision support system 510 is to improve outcomes for PWDs by facilitating communication of clinically relevant "insights" about a PWD's insulin-based therapy to patient systems 504 and/or HCP systems 506 as well as by facilitating communication of therapy related advice from HCP systems 506 to patient systems 504.

In disclosed embodiments, data processing system 502 may be one or more computing platforms configured to process therapy data 112 (FIG. 1) stored at, or received from, therapy management systems 508 and/or clinical decision support system 510. In one embodiment, data processing system 502 may, among other things, include one or more elements of computing platform 100 (FIG. 1), including trained missed-bolus classifier engine 104. In this manner, data processing system 502 may be configured to perform missed-bolus detection for therapy management system 508 and/or clinical decision support system 510.

By way of example, data processing system 502 may perform missed-bolus detection on therapy data 112 (FIG. 1) stored at clinical decision support system 510 and provide one or more reports 120 (FIG. 1) detailing one or more labeled retrospective time periods, as well as one or more metrics for frequency of missed bolus doses. Clinical decision support system 510 may use the data in reports 120 to trigger insights and/or recommendations that it sends to HCP systems 506. Upon HCP system 506 accessing messages from clinical decision support system 510, data from reports 120 may be included in such message or accessible by HCP systems 506. For example, HCP systems 506 requests data to support an insight or recommendation described in a message.

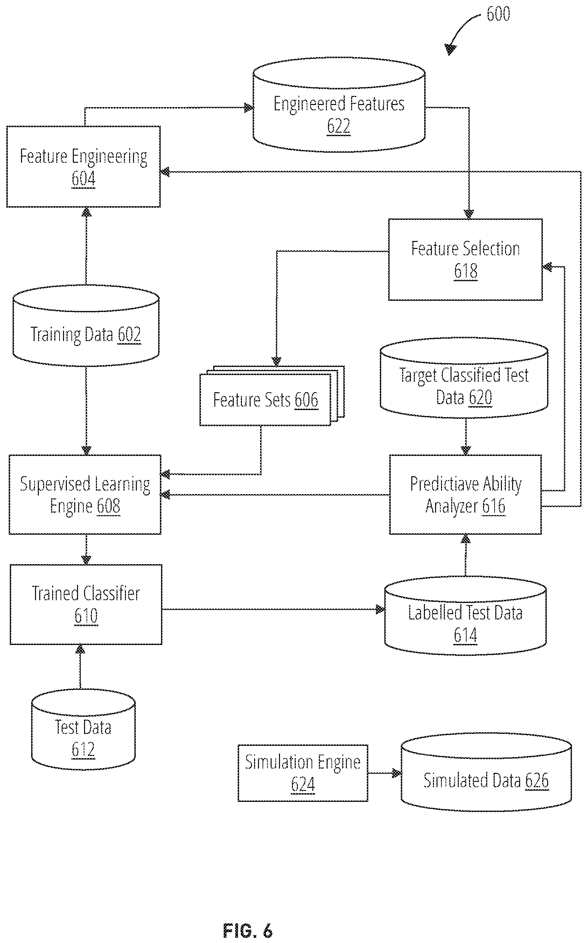

FIG. 6 shows a functional block diagram of a system 600 for training a missed-bolus classifier (such as trained missed-bolus classifier engine 104 in FIG. 1) using machine learning techniques, in accordance with disclosed embodiments.

In a contemplated operation, supervised learning engine 608 trains trained classifier 610 using training data 602 and sets of engineered features (i.e., feature sets 606) selected for model training purposes. In one embodiment, trained classifier 610 is a function or algorithm that detects missed bolus doses. An initial "best guess" may be used for trained classifier 610 which is then continually improved by supervised learning engine 608. In disclosed embodiments, trained classifier 610 and supervised learning engine 608 may implement any suitable supervised learning algorithms and ensemble methods thereof for performing embodiments of the disclosure, including, for example, a logistic regression classifier, a decision tree classifier, an extra tree classifier, an isolation forest classifier, a random forest classifier, and/or a boosting classifiers. Disclosed embodiments may also implement supervised learning algorithm(s) that do not use feature selection, including, for example, one class support vector machine (SVM) without feature selection, and logistic regression without feature selection.

In one embodiment, training data 602 is labeled therapy data associated with one or more PWDs. PWDs may be chosen so they are representative of a desired domain of PWD physiologies, eating behaviors, exercise behaviors, sleeping behaviors, diurnal profile variation, and more.

In disclosed embodiments, feature sets 606 are sub-sets of features engineered (i.e., formed) in the training data 602 and used by supervised learning engine 608 to train any classifier. In one embodiment, feature sets 606 are created using a feature selection process for selecting a subset of features included in a feature domain created using feature engineering techniques. Features in a feature domain may include, for example, one or more of the features identified in Table 1, below:

**Table 1: Examples of Features For a Feature Domain**

| **Name** | **Description** |
|---|---|
| Hour | Temporal hour |
| Minute | Temporal minute |
| Week | Temporal week |
| daysInTherapy | Days from the beginning of therapy |
| isNight | Associated with night |
| isWeekend | Associated with a weekend |
| bolusHour | Hour associated with the time of bolus if there is a bolus, -1 otherwise |
| hoursSinceLastBolus | Hours since the last dose of bolus, -1 if the last dose is not available |
| Filtered EGV | Using Savitsky-Golay filter on linearly interpolated estimated glucose value (EGV) values |
| EGV greater than 350 | Estimated glucose value is greater than 350 mg/dl |
| windowChangeRate | The slope of a line that connects the maximum EGV to the minimum EGV in the 60 minutes moving window |
| EGV rate of change | With different lags 1, 3, 5 |
| Statistics on EGV or EGV rate of change | Median, min/max, median absolute deviation, standard deviation using rolling windows of 5, 10, 30 and 120 minutes. Use for EGV and EGV rate of change |
| mealProb | sum of probabilities over meals, each mealProbability calculated based on normal distribution with meal mean and meal standard deviation from criticalParameterTable settings of in-house PWD simulator |
| bolusInLast60minutes | 1 if there has been a bonus in the last 60 minutes; 0 otherwise |
| bolusInLast120minutes | 1 if there was a bolus in the last 120 minutes; 0 otherwise |
| Filtered egvFirstDerivative | using Savitsky-Golay filter to calculate the first derivative of EGV values |
| Filtered egvSecondDerivative | using Savitsky-Golay filter to calculate the second derivative of EGV values |
| egvFirstDerivativeSign | 1 if D(EGV) > threshold, 0 if -threshold < D(EGV) < threshold, -1 if D(EGV) < - threshold, threshold value needs to be optimized. D stands for the first derivative. |
| egvSecondDerivativeSign | 1 if D²(EGV) > threshold, 0 if -threshold < D²(EGV) < threshold, -1 if D²(EGV) < -threshold, threshold value needs to be optimized. D² stands for the second derivative. |
| egvTriangularShape | an integer between 1 to 7 for various permutation of the combination of the sign of the first and second derivative of EGV values |

Feature sets 606 may be selected from the feature domain using any suitable feature selection technique or combination of techniques for trying features in the feature domain and identifying important features, including, for example, sequential forward feature selection, sequential backward elimination, and tree-based feature selection algorithms.

Labeled test data 614 is test data 612 classified and labeled by a trained classifier 610 during successive iterations of system 600. In one embodiment, the rule for a missed bolus may be a clinically relevant rule (e.g., commonly accepted rule for a "missed bolus" used by HCPs). In one embodiment, the rule is that a bolus is missed where there is not a bolus within substantially 30 minutes of a meal. In another embodiment, the rule is that a bolus is missed where there is not a bolus within a predetermined period of time (e.g., 30 minutes, 45 minutes, 60 minutes) after a recommendation to dose a bolus of insulin is presented to a user, for example, by a therapy management system or therapy management application.

In one embodiment, binary labels (e.g., 0 and 1, or -1 and 1) are used to indicate whether a rule was satisfied.

Labelled test data 614 is the "true" or "target" labels for test data 612. Stated another way, it is the labeling result that is the target for trained classifier 610. Predictive ability analyzer 616 can assess the predictive ability of trained classifier 610 by comparing the labels of the labelled test data 614 that were predicted by the classifier to the true labels in the target classified test data. Any suitable technique for calculating and/or assessing validity of a model may be used by predictive ability analyzer 616, including for example, precision, recall, number of detected events versus number of true events, confusion matrix, area-under-the-free-curve (AUC), and/or receiver operating characteristics (ROC) curve. Techniques such as grid search combined with cross validation, and N-fold cross-validation can be used for hyper parameter tuning of a classifier.

Feature selection 618 receives assessment results from predictive ability analyzer 616 and, in response, changes feature sets 606 to attempt to improve accuracy and/or attempt to simplify feature sets 606. As non-limiting examples, changes to feature sets 606 may include, changing weighting for features of feature sets 606, adding features to feature sets 606 to attempt to improve accuracy of predictions, removing unnecessary features from feature sets 606, and combinations thereof.

Feature engineering 604 receives assessment results from predictive ability analyzer 616 and, in some cases, performs feature engineering techniques to extract new features from test data 612 and add those features to engineered features 622. These new features may be used in the feature selection process by feature selection 618.

In one embodiment, system 600 may include simulation engine 624 configured to generate simulation data 626 from which training data 602 and test data 612 may be obtained. Simulation engine 624 may be configured to simulate insulin therapy scenarios for a variety of PWDs. PWD profiles are created that represent a cross-section of PWDs in terms of characteristics such as physiology (e.g., age, weight, height, complicating health conditions, diurnal profile variation, etc.), lifestyle (e.g., eating behaviors, exercise behaviors, sleeping behaviors, etc.), socio-economic factors (e.g., income, race, geographic location, marriage status, child status, etc.), differences in how PWDs measure and track meal intake, and differences in the operation and quality of insulin delivery systems and components the PWDs use. In one embodiment, simulation engine 624 is configured to model for missing therapy data due to, for example, lost components, failure to input therapy related data, failure to wear a glucose monitor, and lost Bluetooth connection.

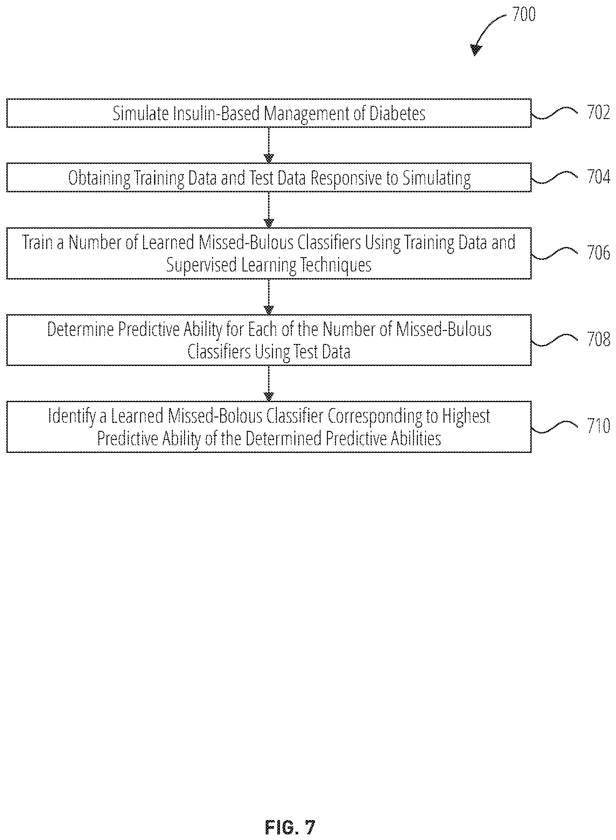

FIG. 7 shows a flowchart for a process 700 for training a missed-bolus classifier, in accordance with disclosed embodiments. Process 700 may be performed, in whole or in part, by embodiments disclosed herein, including by one or more components of system 600.

In operation 702, process 700 simulates insulin-based management of diabetes. In operation 704, process 700 obtains training data and test data responsive to the simulations performed in operation 702. In one embodiment, pre-processing of simulation data obtained from operation 702 may be performed to obtain the training data and test data including the feature sets. In operation 706, process 700 trains a number of missed-bolus classifiers using the training data. In operation 708, process 700 uses the test data to determine a predictive ability for each of the number of missed-bolus classifiers trained in operation 706. In operation 710, process 700 selects a trained missed-bolus classifier corresponding to a highest predictive ability of the predictive abilities determined in operation 708. The trained missed-bolus classifier selected in operation 710 may be used as a trained missed-bolus classifier.

Notably, disclosed embodiments, in whole or in part, may be performed as a series of discrete operations, performed iteratively or reclusively such that the method converges on a final result, or combinations thereof.

In some embodiments, system 600 and process 700 may be used to train a late bolus classifier for performing retrospective late-bolus classification of insulin therapy data, in addition or alternatively, to training a missed-bolus classifier. In one embodiment, a rule for a late bolus may be defined as a bolus dose of insulin was administered after a pre-determined first time threshold and before a pre-determined second time threshold. As an example, a rule for late bolus detection may be that a bolus dose of insulin was administered more than 15 minutes after a meal or after a bolus recommendation but less than 45 minutes after a meal or a bolus recommendation.

In one embodiment, late-bolus classifiers and missed-bolus classifiers may both be used to classify insulin therapy data, and label data as including a missed-bolus event and/or a late-bolus event.

In some embodiments, a rule for missed bolus or late bolus may use a time when blood glucose levels trend lower to detect administration of a bolus dose of insulin. In other words, a decreasing blood glucose level that is indicative of insulin action by the user's body may be used to detect a bolus dose of insulin. A rate of decrease over a period of time that is greater than a rate threshold, or a total decrease of blood glucose level over a period of time that exceeds a total decrease threshold, may be used to determine a time when a bolus dose of insulin was administered in embodiments of this disclosure. A bolus dose may be detected based on a rate of decrease of blood glucose levels or total decrease in blood glucoses, or, alternatively, a bolus may be detected based on either of those in conjunction with a previously recommended dose or meal.

Several non-limiting examples are provided for operation of a missed-bolus classifier and late-bolus classifier in conjunction. The rule used by the late bolus classifier can take into account a time period 15-45 minutes after a recommendation or meal. The rule used by the missed-bolus classifier can take into account a time period 45 minutes or more after a recommendation or meal.

As a non-limiting example, if a bolus dose is recommended at 3PM and a blood glucose trends lower at 3:40, then a late bolus may be detected because 40 minutes is greater than 30 minutes but less than 45 minutes. As another non-limiting example, if a meal is input by a user at 3PM and a blood glucose trends higher at 4PM, then a missed bolus may be detected because 60 minutes is more than 45 minutes.

In one embodiment, the time of a meal may be based on blood glucose levels. More specifically, a time of a meal may be based on when blood glucose levels change by an amount or rise at a rate that is indicative of meal intake. As a non-limiting example, a user may input a meal at 3PM, blood glucose levels may rise at a rate that is greater than a pre-determined rate at 3:18, and then blood glucose trends lower at 4PM. In this example, a late bolus may be detected because 42 minutes is less than 45 minutes.

In embodiments described herein, trends may be based on one or more of a rate of decrease of blood glucose levels, based on a rate at which an increase in blood glucose levels is slowing, measurements of insulin on board, and combinations thereof.

Notably, statistics about missed-bolus detection and late-bolus detection described herein may be reported - e.g., in reports 120 of FIG. 1. For example, a probability that a user will bolus late or miss a bolus may be reported. A clinical decision support (CDS) system, such as CDS system 510 (FIG. 5), may detect that those probabilities exceed a certain threshold and generate a behavioral intervention or action for a patient. As a non-limiting example, a CDS may detect that a user is consistently in the missed and/or late bolus category and recommend to an HCP that the patient be educated about blood glucose levels responses to meal intake and/or insulin in case the patient does not understand when bolus doses should be administered.

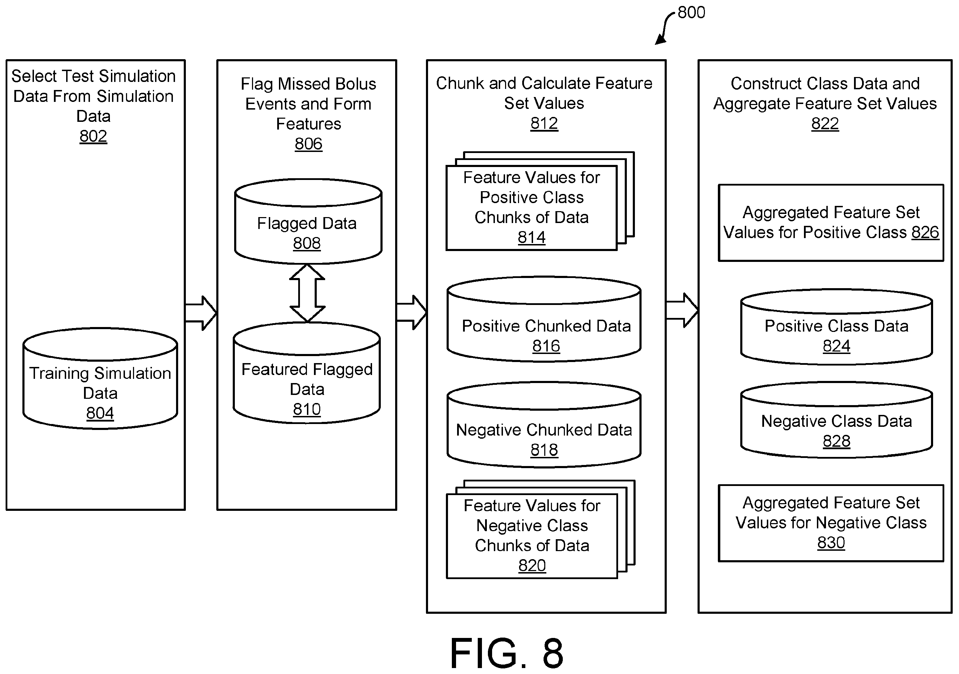

Some disclosed embodiments relate, generally, to obtaining training data and test data, such as training data 602 and test data 612 (FIG. 6), from simulation data such as simulation data 626. FIG. 8 shows a functional block diagram of a data journey 800, for creating training data from simulation data, in accordance with disclosed embodiments. Each stage of data journey 800 is shown as an operational block that describes at least some notable intermediate data elements of data journey 800.

In operational block 802, training simulation data 804 is obtained by selecting part of simulation data (such as simulation data 626 in FIG. 6)) to be training simulation data 804. In one embodiment, 90 days' worth of simulated data is obtained and the first 60 days of simulation data is selected to be training simulation data 804 and the last 30 days of simulation data is selected to be test simulation data.

In operational block 806, each missed bolus event is flagged in the training simulation data 804 to obtain flagged data 808. Notably, each true missed bolus in the simulation data 804 is known for each PWD that was part of a simulation.

In operational block 806, feature engineering techniques are used on flagged data 808 to obtain feature set 810. More specifically, feature engineering techniques are used to form features in flagged data 808 to obtain the feature set 810.

In operational block 812, feature set 810 is chunked to obtain positive chunked data 816 and negative chunked data 818. In various embodiments, a chunk of data is data that is relevant to a class (here a positive class or negative class), and a chunk of data may itself be formed by aggregating sub-units of data. Positive chunked data 816 are chunks of data associated with a positive class (i.e., there is a missed bolus event detected). In one embodiment, positive chunked data 816 may be obtained by aggregating feature set 810 (here, training data) corresponding to the next 60 minutes' worth of observations after each missed bolus event. Further, a 60 minute chunk of therapy data may be formed of therapy data corresponding to 12 instances of consecutive 5 minute observations.

Negative chunked data 818 are chunks of data associated with a negative class (i.e., no missed bolus event detected). In one embodiment, negative chunked data 818 may be obtained by aggregating training data corresponding to a 60 minutes' worth of observations where (1) the observations in the 60 minutes are consecutive in timestamps; (2) chunks of chunked data 818 do not intersect (i.e., the intersection of chunks of chunked data 818 is empty); and (3) no chunks of chunked data 816 and chunks of chunked data 818 intersect (i.e., the intersection of chunks of chunked data 816 and chunked data 818 is empty). In one embodiment, available chunks of data are randomly selected to form chunked data 816 and/or chunked data 818. In one embodiment, a number of chunks selected for chunked data 816 is substantially the same as the number of chunks selected for chunked data 818.

Feature values 814 for positive chunked data 816 and feature values 820 for negative chunked data 818 are obtained for chunks of chunked data 816 and chunks of chunked data 818, respectively, in operational block 812. In various embodiments, feature values may be calculated for a chunk of data or one or more smaller observational units of a chunk of data. For example, feature values may be calculated using therapy data for each of the 5 minute observational units that form a 60 minute chunk of therapy data.

In operational block 822, positive class data 824 and negative class data 828 are obtained, and more specifically, are constructed from chunks of chunked data 816 and chunks of chunked data 818, respectively. In one embodiment, each positive class data 824 is formed by labeling the constituent chunks of data of chunked data 816 with a positive class identifier (e.g., "true" or " 1") and copying the labeled chunks of data into positive class data 824. Similarly, in one embodiment, each negative class data 828 is formed by labeling the constituent chunks of data of chunked data 818 with a negative class identifier (e.g., "false" or "0") and copying the labeled chunks of data to negative class data 828.

In operational block 822, aggregated feature set values 826 for positive class data 824 and aggregated feature set values 830 for negative class data 828 are obtained. In one embodiment, aggregated feature set values 826 for positive class data 824 and aggregated feature set values 830 for negative class data 828 are formed by aggregating feature values 814 for positive chunked data 816 and feature values 820 for negative chunked data 818, respectively, on a feature by feature basis into a single value for the chunk. In various embodiments, any suitable statistical method for aggregating may be used, including, for example, one or more of mean, median, a commercially available aggregate function (e.g., first value).

In various embodiments, training data, such as training data 602 (FIG. 6), may be obtained by combining positive class data 824 and negative class data 828.

In one embodiment, training data obtained as a result of data journey 800 may be characterized as balanced training data, i.e., having a substantially equal number of observations from both classes (i.e., "detected missed bolus" and "no detected missed bolus"). Since the probability of a missed bolus event is lower than the probability of no missed-bolus event, the observations in simulation data 626 (FIG. 6) should (in theory) be imbalanced, more specifically, the negative observations should outweigh the positive observations. So, when obtaining balanced training data, a majority of negative observations may not be considered, and so there is potential for data loss. In one embodiment, any impact of data loss is alleviated by using ensemble or bagging techniques.

In disclosed embodiments, test data, such as test data 612 (FIG. 6), may be obtained in a manner similar to data journey 800 described above, except that the entire set of test simulation data may be chunked. Test data may be imbalanced (i.e., does not have to be balanced). So, in one embodiment, chunks may be formed by applying a rolling window to test simulation data. For example, 60 minute chunks may be formed by travelling across an entire test simulation data in 5 minute by 5 minute observational units, person by person. Features may be formed for each rolling window by aggregating values using suitable statistical methods similar to training data as described above.

In some cases, there may not be enough consecutive observational sub-units available to form a full chunk of data. Using the example of a 60 minute chunk of data formed of 5-minute observational units, after a missed bolus event there may be 6 of the 5-minute observational units and 5 discontinuities (e.g., gaps between observations). So, in one embodiment, a chunk of data is discarded if fewer than a predetermined number of consecutive observational units is available. Each chunk may be assigned a label with an appropriate class identifier to indicate that it is associated with a missed bolus or no missed bolus. In one embodiment, a chunk of data may be assigned a positive class label if any missed bolus event is within plus-minus 5 minutes of a chunk start time, and assigned a negative class label if otherwise.

While embodiments have been described herein with respect to missed-bolus detection and/or late bolus detection, one of ordinary skill in the art would understand that embodiments are equally applicable to missed or late meal detection. For example, detecting a missed and/or late meal after exercise, sleep, and/or a recommendation that a user eat, which could lead to hypoglycemia.

Any characterization in this disclosure of something as "typical," "conventional," or "known" does not necessarily mean that it is disclosed in the prior art or that the discussed aspects are appreciated in the prior art. Nor does it necessarily mean that, in the relevant field, it is widely known, well-understood, or routinely used.

## Claims

1. A computer-implemented method of detecting a missed-bolus dose, comprising:
receiving therapy data associated with an insulin-based management of a person's diabetes over a period of time (202);
identifying a retrospective time period of the period of time (204);
performing, using a trained missed-bolus classifier, a trained missed-bolus classification process on a part of the therapy data that corresponds to the retrospective time period (206), wherein the trained missed-bolus classifier (104) is based on training data and feature sets, wherein the training data comprises positive class training data (824) formed from positive chunked data (816) and negative class training data (828) formed from negative chunked data (818), and wherein the feature sets comprises aggregated feature set values for the positive class training data (826) formed from feature values for positive class chunked data (814) and aggregated feature set values for the negative class training data (830) formed from feature values for negative class chunked data (820);
obtaining a classification result responsive to the performed trained missed-bolus classification process (208); and
assigning a label to the retrospective time period responsive to the classification result (210).

2. The method of claim 1, wherein the obtaining the classification result comprises obtaining a missed-bolus classification result or a no missed-bolus classification result; and/or
wherein the identifying the retrospective time period comprises identifying a substantially two-week time period.

3. The method of claim 1, further comprising calculating a missed-bolus frequency metric responsive to the classification result for the retrospective time period and one or more classification results for one or more other retrospective time periods (212);
and optionally wherein at least one of the one or more other retrospective time periods is earlier than the retrospective time period.

4. The method of claim 1, wherein the receiving the therapy data comprises receiving meal data, blood glucose data, and insulin dosing data associated with the insulin-based management of the person's diabetes over the period of time.

5. The method of claim 1, further comprising:
receiving an identifier for a glucose capture device;
searching for the identifier among a number of identifiers for glucose capture devices that are associated with the trained missed-bolus classification processes; and
selecting the trained missed-bolus classification process responsive to finding the identifier.

6. The method of claim 1, further comprising:
receiving one or more retrospective analysis parameters; and
tuning the trained missed-bolus classification process responsive to the one or more retrospective analysis parameters before preforming the trained missed-bolus classification process on the part of the therapy data that corresponds to the retrospective time period;
and optionally wherein the receiving the one or more retrospective analysis parameters comprises receiving one or more of an identifier for a glucose capture device, a diurnal profile of the person, and meal weighting factors.

7. The method of claim 1, further comprising reporting a missed dose to a system for assisting with clinical decisions responsive to the classification result.

8. A system, comprising:
a data store (110) having stored thereon data, the data comprising therapy data (112) associated with an insulin-based management of a person's diabetes over a period of time;
a trained missed-bolus classifier (104) based on training data and feature sets, wherein the training data comprises positive class training data (824) formed from positive chunked data (816) and negative class training data (828) formed from negative chunked data (818), and wherein the feature sets comprises aggregated feature set values for the positive class training data (826) formed from feature values for positive class chunked data (814) and aggregated feature set values for the negative class training data (830) formed from feature values for negative class chunked data (820)
and
a computing platform operative to be executed as a data processing system responsive to requests to process the therapy data, the data processing system configured to:
identify a retrospective time period of the period of time;
perform, using the trained missed-bolus classifier, a trained missed-bolus classification process on at least a part of the therapy data that corresponds to the retrospective time period;
obtain a classification result responsive to the performed trained missed-bolus classification process; and
assign a label to the retrospective time period responsive to the classification result.

9. The system of claim 8, wherein the trained missed-bolus classification process is a binary classification process.

10. The system of claim 9, wherein the trained missed-bolus classification process returns a true responsive to detecting any missed boluses in the therapy data; and/or
wherein the trained missed-bolus classification process returns a true for each detected missed-bolus in the therapy data.

11. The system of claim 8, wherein the computing platform is configured to identify the retrospective time period by identifying a substantially two-week time period.

12. The system of claim 8, wherein the data processing system is configured to calculate a missed-bolus frequency metric responsive to the classification result for the retrospective time period and one or more classification results for one or more other retrospective periods of time;
and optionally wherein at least one of the one or more other retrospective periods of time is earlier than the retrospective time period.

13. The system of claim 8, wherein the therapy data comprises meal data, blood glucose data, and insulin dosing data associated with the insulin-based management of the person's diabetes over the period of time.

14. The system of claim 13, wherein the data processing system is configured to tune the trained missed-bolus classification process responsive to one or more retrospective analysis parameters before preforming the trained missed-bolus classification process on the part of the therapy data that corresponds to the retrospective time period;
and optionally wherein the one or more retrospective analysis parameters comprise one or more of an identifier for a glucose capture device, a diurnal profile of the person, and meal weighting factors.

15. The system of claim 13, wherein the data processing system is configured to report a missed dose to a system for assisting with clinical decisions responsive to the classification result.

16. The system of claim 8, wherein the data comprises a number of identifiers for glucose capture devices, and wherein the data processing system is configured to:
search the number of identifiers for an identifier of a glucose capture device associated with the therapy data; and
select the trained missed-bolus classification process responsive to finding the identifier.

17. A computer-readable storage medium storing instructions which, when executed by a processor of a computer, cause the computer to perform the method according to any of claims 1 to 7.

18. A computer program product comprising a non-transitory computer-readable storage medium having computer-readable instructions embodied thereon, wherein the computer-readable instructions are adapted to cause a computer running the instructions to perform the method according to any of claims 1 to 7.

## Patentansprüche

1. Ein computerimplementiertes Verfahren zum Detektieren einer verpassten Bolusdose, das Folgendes beinhaltet:
Empfangen von Therapiedaten, die mit einer insulinbasierten Vewaltung eines Diabetes einer Person über einen Zeitraum (202) assoziiert sind;
Identifizieren einer retrospektiven Zeitperiode des Zeitraums (204);
Durchführen, unter Verwendung eines trainierten verpassten-Bolus-Klassifikators, eines trainierten verpassten-Bolus-Klassifikationsprozesses auf einem Teil der Therapiedaten, der der retrospektiven Zeitperiode (206) entspricht, wobei der trainierte verpasste-Bolus-Klassifikator (104) auf Trainingsdaten und Featuresets basiert, wobei die Trainingsdaten Trainigsdaten (8264) der positiven Klasse, die aus positiven kompakten Daten (816) gebildet sind, und Trainingsdaten (828) der negativen Klasse, die aus negativen kompakten Daten (818) gebildet sind, beinhalten und wobei die Featuresets aggregierte Featuresetwerte für die Trainigsdaten (826) der positiven Klasse, gebildet aus Featurewerten für die kompakten Daten (814) der positiven Klasse und aggregierte Featuresetwerte für die Trainigsdaten (830) der negativen Klasse, gebildet aus Featurewerten für die kompakten Daten (820) der negativen Klasse, beinhalten;
Erhalten eines Klassifikationsergebnisses reagierend auf den durchgeführten trainierten verpassten-Bolus-Klassifikationsprozess (208); und
Zuordnen einer Bezeichnung zu der retrospektiven Zeitperiode reagierend auf das Klassifikationsergebnis (210).

2. Verfahren gemäß Anspruch 1, wobei das Erhalten des Klassifikationsergebnisses das Erhalten eines verpassten-Bolus-Klassifikationsergebnisses oder eines nichtverpassten-Bolus-Klassifikationsergebnisses beinhaltet; und/oder
wobei das Identifizieren der retrospektiven Zeitperiode das Identifizieren einer im Wesentlichen zweiwöchigen Zeitperiode beinhaltet.

3. Verfahren gemäß Anspruch 1, das ferner das Berechnen einer verpassten-Bolus-Frequenzmetrik reagierend auf das Klassifikationsergebnis für die retrospektive Zeitperiode und ein oder mehrere Klassifikationsergebnisse für eine oder mehrere andere retrospektive Zeitperioden (212) beinhaltet;
und wobei optional mindestens eine der einen oder der mehreren anderen retrospektiven Zeitperioden früher als die retrospektive Zeitperiode ist.

4. Verfahren gemäß Anspruch 1, wobei das Empfangen der Therapiedaten das Empfangen von Mahldaten, Blutglukosedaten und Insulindosierungsdaten, die mit der insulinbasierten Verwaltung des Diabetes der Person über den Zeitraum assoziiert sind, beinhaltet.

5. Verfahren gemäß Anspruch 1, das ferner Folgendes beinhaltet:
Empfangen eines Identifikators für eine Glukoseerfassungsvorrichtung;
Suchen nach dem Identifikator unter einer Anzahl von Identifikatoren für Glukoseerfassungsvorrichtungen, die mit dem trainierten verpassten-Bolus-Klassifikationsprozess assoziiert sind; und
Auswählen des trainierten verpassten-Bolus-Klassifikationsprozesses reagierend auf das Finden des Identifikators.

6. Verfahren gemäß Anspruch 1, das ferner Folgendes beinhaltet:
Empfangen eines oder mehrerer retrospektiver Analyseparameter; und
Abstimmen des trainierten verpassten-Bolus-Klassifikationsprozesses reagierend auf den einen oder die mehreren retrospektiven Analyseparameter vor dem Durchführen des trainierten verpassten-Bolus-Klassifikationsprozesses auf dem Teil der Therapiedaten, der der retrospektiven Zeitperiode entspricht;
und wobei optional das Empfangen des einen oder der mehreren retrospektiven Analyseparameter das Empfangen eines oder mehrerer eines Identifikators für eine Glukoseerfassungsvorrichtung, eines täglichen Profils der Person und Mahlgewichtungsfaktoren beinhaltet.

7. Verfahren gemäß Anspruch 1, das ferner das Melden einer verpassten Dosis an ein System zum Assistieren mit klinischen Entscheidungen reagierend auf das Klassifikationsergebnis beinhaltet.

8. Ein System, das Folgendes beinhaltet:
einen Datenspeicher (110), der darauf gespeichert Daten aufweist, wobei die Daten Therapiedaten (112) beinhalten, die mit einer insulinbasierten Verwaltung des Diabetes einer Person über einen Zeitraum assoziiert sind;
einen trainierten verpassten-Bolus-Klassifikator (104) basierend auf Trainingsdaten und Featuresets, wobei die Trainingsdaten Trainigsdaten (824) der positiven Klasse, die aus positiven kompakten Daten (816) gebildet sind, und Trainingsdaten (828) der negativen Klasse, die aus negativen kompakten Daten (818) gebildet sind, beinhalten und wobei die Featuresets aggregierte Featuresetwerte für die Trainigsdaten (826) der positiven Klasse, gebildet aus Featurewerten für die kompakten Daten (814) der positiven Klasse und aggregierte Featuresetwerte für die Trainigsdaten (830) der negativen Klasse, gebildet aus Featurewerten für die kompakten Daten (820) der negativen Klasse, beinhaltet
und
eine Rechenplattform, die operativ ist, um als ein Datenverarbeitungssystem reagierend auf Anfragen zum Verarbeiten der Therapiedaten ausgeführt zu werden, wobei das Datenverarbeitungssystem für Folgendes konfiguriert ist:
Identifizieren einer retrospektiven Zeitperiode des Zeitraums;
Durchführen, unter Verwendung des trainierten verpassten-Bolus-Klassifikators, eines trainierten verpassten-Bolus-Klassifikationsprozesses auf mindestens einem Teil der Therapiedaten, der der retrospektiven Zeitperiode entspricht;
Erhalten eines Klassifikationsergebnisses reagierend auf den durchgeführten trainierten verpassten-Bolus-Klassifikationsprozess; und
Zuordnen einer Bezeichnung zu der retrospektiven Zeitperiode reagierend auf das Klassifikationsergebnis.

9. System gemäß Anspruch 8, wobei der trainierte verpasste-Bolus-Klassifikationsprozess ein binärer Klassifikationsprozess ist.

10. System gemäß Anspruch 9, wobei der trainierte verpasste-Bolus-Klassifikationsprozess reagierend auf das Detektieren jeglicher verpasster Boli in den Therapiedaten ein wahr wiedergibt; und/oder
wobei der trainierte verpasste-Bolus-Klassifikationsprozess ein wahr für jeden detektierten verpassten Bolus in den Therapiedaten wiedergibt.

11. System gemäß Anspruch 8, wobei die Rechenplattform konfiguriert ist, um die retrospektive Zeitperiode durch das Identifizieren einer im Wesentlichen zweiwöchigen Zeitperiode zu identifizieren.

12. System gemäß Anspruch 8, wobei das Datenverarbeitungssystem konfiguriert ist, um eine verpasste-Bolus-Frequenzmetrik reagierend auf das Klassifikationsergebnis für die retrospektive Zeitperiode und ein oder mehrere Klassifikationsergebnisse für eine oder mehrere retrospektive Zeitperioden zu berechnen;
und wobei optional mindestens eine der einen oder mehreren anderen retrospektiven Zeitperioden früher als die retrospektive Zeitperiode ist.

13. System gemäß Anspruch 8, wobei die Therapiedaten Mahldaten, Blutglukosedaten und Insulindosierungsdaten, die mit der insulinbasierten Verwaltung des Diabetes der Person über den Zeitraum assoziiert sind, beinhaltet.

14. System gemäß Anspruch 13, wobei das Datenverarbeitungssystem konfiguriert ist, um den trainierten verpassten-Bolus-Klassifikationsprozess reagierend auf einen oder mehrere retrospektive Analyseparameter abzustimmen, bevor der trainierte verpasste-Bolus-Klassifikationsprozess auf dem Teil der Therapiedaten durchgeführt wird, der der retrospektiven Zeitperiode entspricht;
und wobei optional der eine oder die mehreren retrospektiven Analyseparameter eines oder mehrere von einem Identifikator für eine Glukoseerfassungsvorrichtung, eines täglichen Profils der Person und Mahlgewichtungsfaktoren beinhaltet.

15. System gemäß Anspruch 13, wobei das Datenverarbeitungssystem konfiguriert ist, um eine verpasste Dosis an ein System zum Assistieren mit klinischen Entscheidungen reagierend auf das Klassifikationsergebnis zu melden.

16. System gemäß Anspruch 8, wobei die Daten eine Anzahl von Identifikatoren für Glukoseerfassungsvorrichtungen beinhalten und wobei das Datenverarbeitungssystem für Folgendes konfiguriert ist:
Durchsuchen der Anzahl von Identifikatoren nach einem Identifikator einer Glukoseerfassungsvorrichtung, die mit den Therapiedaten assoziiert ist; und
Auswählen des trainierten verpassten-Bolus-Klassifikationsprozesses reagierend auf das Finden des Identifikators.

17. Ein durch einen Computer lesbares Speichermedium, das Anweisungen speichert, die, wenn sie durch einen Prozessor eines Computers durchgeführt werden, bewirken, dass der Computer das Verfahren gemäß einem der Ansprüche 1 bis 7 durchführt.

18. Ein Computerprogrammprodukt, das ein nicht transitorisches durch einen Computer lesbares Speichermedium mit darauf enthaltenen durch einen Computer lesbaren Anweisungen beinhaltet, wobei die durch einen Computer lesbaren Anweisungen angepasst sind, um zu bewirken, dass ein Computer, der die Anweisungen laufen lässt, das Verfahren gemäß einem der Ansprüche 1 bis 7 durchführt.

## Revendications

1. Un procédé de détection d'une dose de bolus manqué mis en œuvre par ordinateur, comprenant :
le fait de recevoir des données thérapeutiques associées à une gestion basée sur l'insuline du diabète d'une personne sur un laps de temps (202) ;
le fait d'identifier un laps de temps rétrospectif du laps de temps (204) ;
le fait d'effectuer, à l'aide d'un classificateur de bolus manqué entraîné, un processus de classification de bolus manqué entraîné sur une partie des données thérapeutiques qui correspond au laps de temps rétrospectif (206), où le classificateur de bolus manqué entraîné (104) est basé sur des données d'entraînement et des ensembles d'attributs, où les données d'entraînement comprennent des données d'entraînement de classe positive (824) formées à partir de données mémorisées par bloc positives (816) et des données d'entraînement de classe négative (828) formées à partir de données mémorisées par bloc négatives (818), et où les ensembles d'attributs comprennent des valeurs d'ensembles d'attributs agrégées pour les données d'entraînement de classe positive (826) formées à partir de valeurs d'attributs pour des données mémorisées par bloc de classe positive (814) et des valeurs d'ensembles d'attributs agrégées pour les données d'entraînement de classe négative (830) formées à partir de valeurs d'attributs pour des données mémorisées par bloc de classe négative (820) ;
le fait d'obtenir un résultat de classification en réponse au processus de classification de bolus manqué entraîné effectué (208) ; et
le fait d'attribuer une étiquette au laps de temps rétrospectif en réponse au résultat de classification (210).

2. Le procédé de la revendication 1, où le fait d'obtenir le résultat de classification comprend le fait d'obtenir un résultat de classification « bolus manqué » ou un résultat de classification « pas de bolus manqué » ; et/ou bien
où le fait d'identifier le laps de temps rétrospectif comprend le fait d'identifier un laps de temps de substantiellement deux semaines.

3. Le procédé de la revendication 1, comprenant en sus le fait de calculer une mesure de fréquence de bolus manqué en réponse au résultat de classification pour le laps de temps rétrospectif et à un ou plusieurs résultats de classification pour un ou plusieurs autres laps de temps rétrospectifs (212) ;
et facultativement où au moins un laps de temps des un ou plusieurs autres laps de temps rétrospectifs est antérieur au laps de temps rétrospectif.

4. Le procédé de la revendication 1, où le fait de recevoir les données thérapeutiques comprend le fait de recevoir des données sur les repas, des données sur le glucose dans le sang, et des données sur le dosage d'insuline associées à la gestion basée sur l'insuline du diabète de la personne sur le laps de temps.

5. Le procédé de la revendication 1, comprenant en sus :
le fait de recevoir un identificateur pour un dispositif de capture de glucose ;
le fait de rechercher l'identificateur parmi un nombre d'identificateurs pour des dispositifs de capture de glucose qui sont associés aux processus de classification de bolus manqué entraînés ; et
le fait de sélectionner le processus de classification de bolus manqué entraîné en réponse au fait de trouver l'identificateur.

6. Le procédé de la revendication 1, comprenant en sus :
le fait de recevoir un ou plusieurs paramètres d'analyse rétrospective ; et
le fait de régler le processus de classification de bolus manqué entraîné en réponse aux un ou plusieurs paramètres d'analyse rétrospective avant d'effectuer le processus de classification de bolus manqué entraîné sur la partie des données thérapeutiques qui correspond au laps de temps rétrospectif ;
et facultativement où le fait de recevoir les un ou plusieurs paramètres d'analyse rétrospective comprend le fait de recevoir un ou plusieurs éléments parmi un identificateur pour un dispositif de capture de glucose, un profil diurne de la personne, et des facteurs de pondération de repas.

7. Le procédé de la revendication 1, comprenant en sus le fait de rapporter une dose manquée à un système pour aider avec des décisions cliniques en réponse au résultat de classification.

8. Un système, comprenant :
une unité de stockage de données (110) sur laquelle sont stockées des données, les données comprenant des données thérapeutiques (112) associées à une gestion basée sur l'insuline du diabète d'une personne sur un laps de temps ;
un classificateur de bolus manqué entraîné (104) basé sur des données d'entraînement et des ensembles d'attributs, où les données d'entraînement comprennent des données d'entraînement de classe positive (824) formées à partir de données mémorisées par bloc positives (816) et des données d'entraînement de classe négative (828) formées à partir de données mémorisées par bloc négatives (818), et où les ensembles d'attributs comprennent des valeurs d'ensembles d'attributs agrégées pour les données d'entraînement de classe positive (826) formées à partir de valeurs d'attributs pour des données mémorisées par bloc de classe positive (814) et des valeurs d'ensembles d'attributs agrégées pour les données d'entraînement de classe négative (830) formées à partir de valeurs d'attributs pour des données mémorisées par bloc de classe négative (820) ;
et
une plate-forme informatique opérationnelle afin d'être exécutée en tant que système de traitement de données en réponse à des demandes de traitement des données thérapeutiques, le système de traitement de données étant configuré afin :
d'identifier un laps de temps rétrospectif du laps de temps ;
d'effectuer, à l'aide du classificateur de bolus manqué entraîné, un processus de classification de bolus manqué entraîné sur au moins une partie des données thérapeutiques qui correspond au laps de temps rétrospectif ;
d'obtenir un résultat de classification en réponse au processus de classification de bolus manqué entraîné effectué ; et
d'attribuer une étiquette au laps de temps rétrospectif en réponse au résultat de classification.

9. Le système de la revendication 8, où le processus de classification de bolus manqué entraîné est un processus de classification binaire.

10. Le système de la revendication 9, où le processus de classification de bolus manqué entraîné renvoie un vrai en réponse à la détection d'un nombre quel qu'il soit de bolus manqués dans les données thérapeutiques ; et/ou bien
où le processus de classification de bolus manqué entraîné renvoie un vrai pour chaque bolus manqué détecté dans les données thérapeutiques.

11. Le système de la revendication 8, où la plate-forme informatique est configurée afin d'identifier le laps de temps rétrospectif par identification d'un laps de temps de substantiellement deux semaines.

12. Le système de la revendication 8, où le système de traitement de données est configuré afin de calculer une mesure de fréquence de bolus manqué en réponse au résultat de classification pour le laps de temps rétrospectif et à un ou plusieurs résultats de classification pour un ou plusieurs autres laps de temps rétrospectifs ;
et facultativement où au moins un laps de temps des un ou plusieurs autres laps de temps rétrospectifs est antérieur au laps de temps rétrospectif.

13. Le système de la revendication 8, où les données thérapeutiques comprennent des données sur les repas, des données sur le glucose dans le sang, et des données sur le dosage d'insuline associées à la gestion basée sur l'insuline du diabète de la personne sur le laps de temps.

14. Le système de la revendication 13, où le système de traitement de données est configuré afin de régler le processus de classification de bolus manqué entraîné en réponse à un ou plusieurs paramètres d'analyse rétrospective avant d'effectuer le processus de classification de bolus manqué entraîné sur la partie des données thérapeutiques qui correspond au laps de temps rétrospectif ;
et facultativement où les un ou plusieurs paramètres d'analyse rétrospective comprennent un ou plusieurs éléments parmi un identificateur pour un dispositif de capture de glucose, un profil diurne de la personne, et des facteurs de pondération de repas.

15. Le système de la revendication 13, où le système de traitement de données est configuré afin de rapporter une dose manquée à un système pour aider avec des décisions cliniques en réponse au résultat de classification.

16. Le système de la revendication 8, où les données comprennent un nombre d'identificateurs pour des dispositifs de capture de glucose, et où le système de traitement de données est configuré afin :
de rechercher dans le nombre d'identificateurs un identificateur d'un dispositif de capture de glucose associé aux données thérapeutiques ; et
de sélectionner le processus de classification de bolus manqué entraîné en réponse au fait de trouver l'identificateur.

17. Un support de stockage lisible par ordinateur stockant des instructions qui, lorsqu'elles sont exécutées par un processeur d'un ordinateur, amènent l'ordinateur à effectuer le procédé selon n'importe lesquelles des revendications 1 à 7.

18. Un produit-programme d'ordinateur comprenant un support de stockage lisible par ordinateur non transitoire sur lequel sont incorporées des instructions lisibles par ordinateur, où les instructions lisibles par ordinateur sont conçues afin d'amener un ordinateur exécutant les instructions à effectuer le procédé selon n'importe lesquelles des revendications 1 à 7.
